# EUROPÄISCHE PATENTSCHRIFT

(11) **EP 1 099 686 B1**
(45) Veröffentlichungstag und Bekanntmachung des Hinweises auf die Patenterteilung: **04.04.2007**
(21) Anmeldenummer: 00123357.6
(22) Anmeldetag: 30.10.2000
(51) Int. Cl.: C07C 209/36

(54) **Verfahren zur Herstellung von Trifluormethylanilinen**
Process for the preparation of trifluormethylanilines
Procédé pour la préparation de trifluorméthylanilines

(30) Priorität: 10.11.1999 DE 19954014
(43) Veröffentlichungstag der Anmeldung: 16.05.2001
(73) Patentinhaber: Saltigo GmbH, 51369 Leverkusen (DE)
(72) Erfinder: Marhold, Albrecht, Dr., 51373 Leverkusen (DE); Baumann, Käthe, Dr., 50672 Köln (DE)
(74) Vertreter: Wichmann, Birgid

(56) Entgegenhaltungen:
- FR-A- 745 293
- GB-A- 1 111 904
- US-A- 4 393 257
- US-A- 4 582 935

## Beschreibung

Die vorliegende Erfindung betrifft ein verbessertes Verfahren zur Herstellung von Trifluormethylanilinen, ausgehend von Benzotrichloriden.

Trifluormethylaniline sind wichtige Zwischenprodukte zur Herstellung von pharmazeutischen und agrochemischen Wirkstoffen, beispielsweise von Herbiziden, Insektiziden, infektionshemmenden Wirkstoffen und Desinfektionsmitteln. Es besteht deshalb das Bedürfnis nach einem Verfahren zur Herstellung von Trifluormethylanilinen, mit dem technische Mengen auf einfache und kostengünstige Weise und in guten Ausbeuten und Reinheiten hergestellt werden können.

Es sind schon einige Verfahren zur Herstellung von Trifluormethylanilinen bekannt geworden. Diese sind jedoch alle für eine Ausübung in technischem Maßstab nicht geeignet oder mit sonstigen gravierenden Nachteilen verbunden.

Von der Verwendung von Schwefeltetrafluorid zur Herstellung von Trifluormethylanilinen im technischen Maßstab (siehe J. Org. Chem. 26, 1477 (1961) und 27, 1406 (1962)) ist wegen dessen extremer Toxizität Abstand zu nehmen.

Bei der Einführung der Trifluormethylgruppe mittels Natriumtrifluoracetat in Gegenwart von stöchiometrischen Mengen Kupfer(I)-iodid (siehe J. Chem. Soc. Perk. Trans. 1, **1988**, 921) werden teure Reagenzien benötigt, und es entstehen Probleme bei der Entsorgung von Kupfersalzen.

Die Fluorierung von Tribrommethylnitrobenzol mit Antimontrifluorid und die anschließende Reduktion des erhaltenen Trifluormethylnitrobenzols mittels Zinnchlorid (siehe J. Am. Chem. Soc. 69, 2346 (1947)) erfordert ebenfalls teure Reagenzien und hohen Aufwand für ökologische Maßnahmen.

Die Umsetzung von Chlorbenzotrifluorid mit Ammoniak und Kupfer(I)-chlorid (siehe J. Org. Chem. 44, 4731 (1979)) erfordert drastische Reaktionsbedingungen und liefert nur geringe Ausbeuten.

Wenn man Benzotrifluorid nitriert und das dabei erhältliche Nitrobenzotrifluorid reduziert, erhält man große Mengen 3-Trifluormethylanilin (um 90 %), etwas 2-Trifluormethylanilin (um 9 %) und nur wenig 4-Trifluormethylanilin (um 1 %) (siehe Synthesis 11, 1087 (1992)). Ähnlich sind die Verhältnisse bei der Nitrierung von substituierten Benzotrifluoriden. Bekannt sind lediglich Verfahren zur Herstellung von 3-Chlor-6-nitro-benzotrifluorid und 3-Fluor-6-nitro-benzotrifluorid (US-A 2 086 029), bei denen nur "to a small extent" 3-Chlor-4-nitro-benzotrifluorid und 3-Fluor-4-nitrobenzotrifluorid anfallen. Häufig sind jedoch die 4-Trifluormethylaniline die gesuchteren Verbindungen.

Es ist schon lange bekannt, Trifluormethylnitrobenzol katalytisch zum Trifluormethylanilin zu reduzieren (siehe J. Org. Chem. 26, 1477 (1964)), jedoch fehlt, wie oben dargelegt, ein technisch günstiger Zugang zu 4-Trifluormethylnitrobenzol.

Ein neueres Verfahren zur Herstellung von Trifluormethylanilin geht von Trichlormethylphenylisocyanat aus, das zunächst mit wasserfreier Flusssäure und dann mit Wasser umgesetzt wird. Man erhält so Trifluormethylanilinhydrofluorid, aus dem das freie Anilin mit einer Base freizusetzen ist (siehe EP-A 639 556). Das Ausgangsisocyanat muss vorher hergestellt werden, z.B. durch Chlorierung von Methylphenylisocyanat. Nachteilig bei diesem Verfahren ist insbesondere seine Vielstufigkeit.

Ein anderes neueres Verfahren geht von Chlorbenzotrifluorid aus, das mit Ammoniak oder Amin in Gegenwart eines Palladiumkatalysators, eines Co-Katalysators und einer starken Base umgesetzt wird (siehe EP-A 846 676). Neben der Komplexität des Verfahrens ist dabei nachteilig, dass auch beim Einsatz eines isomerenreinen Ausgangsmaterials ein Produkt entsteht, das ein Isomerengemisch darstellt.

Es wurde nun ein Verfahren zur Herstellung von Trifluormethylanilinen der Formel (I) gefunden 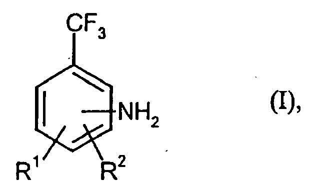 in der
- R¹: für Wasserstoff, Fluor, Chlor, Brom, Methyl, Monochlormethyl, Dichlormethyl oder Formyl und
- R²: für Wasserstoff, Fluor oder Chlor stehen
und sich im Falle R¹ = R² = Wasserstoff die Aminogruppe in para-Position zur Trifluormethylgruppe befindet,
wobei man ein Benzotrichlorid der Formel (II) 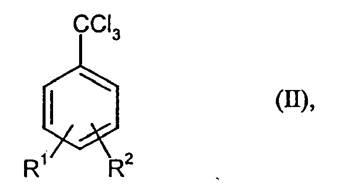 in der
R¹ und R² die bei Formel (I) angegebene Bedeutung haben,
nitriert, bei den so erhältlichen Nitrobenzotrichloriden die Trichlormethylgruppen durch Umsetzung mit wasserfreier Fluorwasserstoffsäure in Trifluormethylgruppen überführt und abschließend die Nitrogruppen reduziert,
dadurch gekennzeichnet, dass die erhaltenen Verbindungen der Formel (1) mindestens 19 % Trifluormethylaniline enthalten, worin sich die Aminogruppe in para-Position zur Trifluormethylgruppe befindet.

In den Formeln (I) und (II) steht R¹ vorzugsweise für Wasserstoff, Fluor oder Chlor und R² vorzugsweise für Wasserstoff oder Chlor.

In die erste Stufe des erfindungsgemäßen Verfahrens, der Nitrierung von Benzotrichloriden der Formel (II), setzt man vorzugsweise Gemische aus Salpetersäure und Schwefelsäure als Nitriermittel ein, wobei die Schwefelsäure vorzugsweise mindestens 96 %-ig ist und es sich bei der Salpetersäure um sog. rauchende Salpetersäure mit Gehalten um 100 % HNO₃ handelt. Die Salpetersäure wird vorzugsweise im Überschuss eingesetzt, beispielsweise 1,2 bis 5 mol pro Mol Benzotrichlorid der Formel (II). Vorzugsweise liegt diese Menge bei 1,8 bis 2,5 mol. Das Gewichtsverhältnis von Salpetersäure zu Schwefelsäure kann beispielsweise 0,5 bis 2:1 betragen. Man kann auch zuerst nur Salpetersäure und dann Schwefelsäure zugeben. Eine Zugabe von Schwefelsäure ohne Vorlage von oder gleichzeitiger Zugabe mit Salpetersäure zu Benzotrichloriden der Formel (II) ist zu vermeiden.

Die Nitrierung kann z.B. bei Temperaturen im Bereich -15 bis +50°C durchgeführt werden. Vorzugsweise arbeitet man im Bereich von -10 bis +35°C. Gegebenenfalls kann man in Gegenwart eines inerten Lösungsmittels nitrieren. Geeignet sind beispielsweise Dichlormethan und 1,2-Dichlorethan.

Die Nitrierung kann batch-weise diskontinuierlich oder auch kontinuierlich z.B. in einen Rohrreaktor erfolgen. Ebenfalls ist der Einsatz von Bündelreaktoren oder Mikroreaktoren möglich.

Die Aufarbeitung des nach der Nitrierung vorliegenden Reaktionsgemisches kann z.B. so erfolgen, dass man es auf Eis austrägt, mit einem inerten organischen Lösungsmittel extrahiert, die vereinigten Extrakte säurefrei wäscht (z.B. mit Wasser und/oder wässriger Natriumhydrogencarbonatlösung), trocknet und das Extraktionsmittel abzieht.

In die zweite Stufe des erfindungsgemäßen Verfahrens, die Umsetzung mit wasserfreier Fluorwasserstoffsäure, kann man das in der ersten Stufe erhaltene Gemisch isomerer Nitrobenzotrichloride einsetzen. Man kann aber auch vorher die Isomere trennen, z.B. durch Destillation oder Kristallisation, und isomerenreine Nitrobenzotrichloride mit wasserfreier Fluorwasserstoffsäure umsetzen.

Pro Mol Nitrobenzotrichlorid kann man beispeilsweise 3 bis 50 mol wasserfreie Fluorwasserstoffsäure einsetzen, wobei das im Handel unter der Bezeichnung "wasserfreie Fluorwasserstoffsäure" erhältliche Material ausreichend wasserfrei ist.

Die Fluorierung kann z.B. bei Temperaturen im Bereich 0 bis 180°C und Drucken im Bereich 1 bis 50 bar durchgeführt werden. Bevorzugt arbeitet man bei 10 bis 160°C und 1 bis 30 bar. Gegebenenfalls kann man in Gegenwart eines Katalysators und/oder eines inerten Lösungsmittels arbeiten. Als Katalysator kann etwa Bortrifluorid, Titantetrachlorid und Antimonpentachlorid und -fluorid verwendet werden, als Lösungsmittel Dichlormethan.

Man kann die wasserfreie Fluorwasserstoffsäure vorlegen und das Nitrobenzotrichlorid zudosieren oder umgekehrt verfahren. Es ist vorteilhaft, die Fluorwasserstoffsäure und das Nitrobenzotrichlorid im Rahmen der oben genannten Temperaturbereiche bei relativ niedrigen Temperaturen, z.B. bis zu 50°C, zu vereinen und danach die Temperatur zu erhöhen. Nach beendeter Reaktion kann man das Reaktionsgemisch mit einem geeigneten Lösungsmittel versetzen, z.B. mit Dichlormethan, die organische Phase abtrennen, mit Wasser waschen, trocknen und einengen oder über eine Brücke destillieren.

Man kann die Fluorierung auch in der Gasphase durchführen, wobei die Reaktionstemperatur beispielsweise 200 bis 450°C betragen kann.

Wenn man möglichst isomerenreine Trifluormethylaniline der Formel (I) herstellen möchte, ist es vorteilhaft, die Isomerentrennung auf der Stufe der Nitrobenzotrifluoride vorzunehmen. Beispielsweise kann man diese Isomerentrennung durch Feindestillation oder eine Kombination aus Feindestillation und Kristallisation vornehmen. Bei der Feindestillation wendet man vorzugsweise solche Drucke an, dass der Sumpf nicht auf Temperaturen von über 200°C erhitzt werden muß. Es hat sich als vorteilhaft erwiesen, die Feindestillation bei Drucken im Bereich 50 bis 150 mbar und bei Sumpftemperaturen im Bereich von 110 bis 180°C durchzuführen. Eine Kombination von Feindestillation und Kristallisation kann man z.B. so durchführen, dass man zunächst ein mindestens 80 % reines Produkt durch Feindestillation isoliert und aus diesem durch Kristallisation noch reinere Produkte erhält. Die Mutterlauge aus der Kristallisation kann in die Feindestillation zurückgeführt werden. Auf diese Weise kann man z.B. 4-Nitrobenzotrifluorid in einer Reinheit von über 99 % erhalten, aus dem dann in der dritten Stufe ähnlich reines 4-Trifluormethylanilin herstellbar ist. Die Kristallisation kann man beispielsweise einfach durch Abkühlen oder durch Zugabe eines Lösungsmittels durchführen, indem die Trifluormethylaniline schwer löslich sind. Man kann gegebenenfalls das durch Kristallisation erhaltene Produkt durch eine Umkristallisation weiter reinigen.

Die dritte Stufe des erfindungsgemäßen Verfahrens, die Reduktion, kann man auf an sich bekannte Weise durchführen, beispielsweise als chemische Reduktion unter Verwendung von beispielsweise Hydrazinhydrat, Ammoniumformiat, Triethylamin/Ameisensäure, Zinn(II)-chlorid, Eisen, Natriumsulfid oder Natriumhydrogensulfid als Reduktionsmittel.

Bevorzugt ist jedoch die katalytische Reduktion mit Wasserstoff in Gegenwart von Katalysatoren, z.B. Raney-Nickel oder Palladium enthaltenden Katalysatoren. Die katalytische Reduktion kann man z.B. bei Temperaturen im Bereich von 20 bis 100°C, Drucken im Bereich von 1 bis 50 bar und gegebenenfalls in Gegenwart von Lösungsmitteln wie Alkoholen oder Estern, vorzugsweise C₁-C₄-Alkylalkoholen oder deren Acetaten, durchführen.

Bei der katalytischen Hydrierung findet i.a. keine nennenswerte Isomerisierung statt, d.h. man erhält i.a. isomerenreine oder weitgehend isomerenreine Trifluonmethylaniline, wenn man ein entsprechendes isomerenreines oder weitgehend isomerenreines Nitrobenzotrifluorid eingesetzt hat. Wenn man Nitrobenzotrifluoride als Isomerengemische eingesetzt hat, erhält man i.a. Trifluormethylanilin-Isomerengemische mit gleicher oder ähnlicher Isomerenverteilung.

Wenn man isomerenreine Trifluormethylaniline erhalten möchte, ist es zweckmäßig, die Isomerentrennung vor der Reduktionsstufe durchzuführen.

Das nach einer katalytischen Reduktion vorliegende Gemisch kann man auf einfache Weise aufarbeiten, z.B. indem man es filtriert und anschließend das Filtrat einengt. Nach einer chemischen Reduktion kann man aufarbeiten, wie es für derartige Reaktionen im Prinzip bekannt ist.

Mit dem erfindungsgemäßen Verfahren kann man in einfacher Weise, mit wenigen Reaktionsstufen, in gängigen Apparaten und mit guten Ausbeuten und Reinheiten Trifluormethylaniline herstellen. Es lassen sich auch Trifluormethylaniline mit höheren Gehalten an 4-Isomeren herstellen als gemäß dem Stand der Technik. Problematische Reagenzien wie Schwefeltetrafluorid und teure Reagenzien wie Bromide oder Iodide müssen nicht eingesetzt werden. Besondere ökologische Probleme treten nicht auf.

Dies ist überraschend, denn ausgehend von Benzotrifluoriden (statt Benzotrichloriden) erhält man schlechtere Ergebnisse, was sich z.B. in einem hohen Anteil an 3-Isomeren manifestiert (um 90 %). Gerade die Trennung der 3- von den 4-Isomeren ist wegen deren geringen Siedepunktsunterschieden besonders aufwendig. Die beim erfindungsgemäßen Verfahren anfallenden, wesentlich geringeren Mengen an 3-Isomeren können, nach Abtrennung der anderen Isomeren auf der Stufe der Nitroverbindungen, ebenfalls sehr rein erhalten und als Zwischenprodukte für Pharma- und Agrowirkstoffe verwendet werden. Es ist auch möglich, auf der Stufe der Nitrobenzotrichloride unerwünschte Isomere abzutrennen und in die entsprechenden Nitrobenzoylchloride überzuführen, die aus den Benzotrifluoriden nicht zugänglich sind. Erfindungsgemäß wird auch ein Zugang zu bisher nicht oder nur sehr schwierig erhältlichen Verbindungen geschaffen wie 3,4-Dichlor-5-trifluormethylanilin, 3,4-Dichlor-2-trifluormethylanilin und den entsprechenden Nitroverbindungen.

Wenn man erfindungsgemäß arbeitet, kann man ausgehend von Benzotrichloriden der Formel (II) Produkte erhalten, die mindestens 19 % 4-Trifluormethylaniline (Formel (I), NH₂ in para-Stellung zur CF₃-Gruppe) enthalten.

### Beispiele

### Beispiel 1

Zu einem Gemisch aus 96 gew.-%-iger Schwefelsäure (564 g) und 100 %-iger rauchender Salpetersäure (564 g) wurde bei -7 bis 0°C Benzotrichlorid (1000 g) im Verlaufe von 4 Stunden zugetropft. Danach wurde das Reaktionsgemisch zunächst für 30 Minuten auf 10°C erwärmt, dann auf Eis ausgetragen und schließlich mit Dichlormethan extrahiert (3 x 500 ml). Die vereinigten organischen Phasen wurden mit Wasser (500 ml), gesättigter wässriger Natriumhydrogencarbonatlösung (3 x 500 ml) und wässriger Natriumchloridlösung (500 ml) gewaschen, über Magnesiumsulfat getrocknet und eingedampft. So wurde ein flüssiges Produkt (1207 g, Reinheit 98,5 %, 96,7 % der Theorie) erhalten, das 80,6 % 3-Nitrobenzotrichlorid und 19,4 % 4-Nitrobenzotrichlorid enthielt.

### Beispiel 2

Zu einem Gemisch aus 96 gew.-%-iger Schwefelsäure (2,26 kg) und 100 %-iger rauchender Salpetersäure (2,26 kg) wurde bei 0°C Benzotrichlorid (4000 g) im Verlaufe von 4 Stunden zugetropft. Danach wurde das Reaktionsgemisch zunächst für 30 Minuten auf 10°C angewärmt, dann auf Eis ausgetragen und schließlich mit Dichlormethan extrahiert (3 x 200 ml). Die vereinigten organischen Phasen wurden mit Wasser (2000ml), gesättigter wässriger Natriumhydrogencarbonatlösung (3 x 2000 ml) und wässriger Natriumchloridlösung (2000 ml) gewaschen, über Magnesiumsulfat getrocknet und eingedampft. So wurde ein flüssiges Produkt (4,4kg, Reinheit 94,6 %, 84,6 % der Theorie) erhalten, das 75 % 3-Nitrobenzotrichlorid und 25 % 4-Nitrobenzotrichlorid enthielt.

### Beispiel 3

Zu einem Gemisch aus 96 gew.-%-iger Schwefelsäure (48 g) und 100 %-iger rauchender Salpetersäure (48 g) wurde bei 10 bis 20°C 3-Chlorbenzotrichlorid (100 g) im Verlaufe von 15 Minuten zugetropft. Danach wurde das Reaktionsgemisch zunächst 2 Stunden bei 20 bis 30°C nachgerührt, dann auf Eis ausgetragen und mit Dichlormethan extrahiert (3 x 100 ml). Die vereinigten organischen Phasen wurden mit Wasser (100 ml), gesättigter wässriger Natriumhydrogencarbonatlösung (3 x 100 ml) und wässriger Natriumchloridlösung (100 ml) gewaschen, über Magnesiumsulfat getrocknet und eingedampft. Es wurde ein Produkt erhalten (115,63 g, Reinheit 100 %, 96,7 % der Theorie), das 26,3 % 3-Chlor-4-nitro-benzotrichlorid und 73,7 % 3-Chlor-6-nitro-benzotrichlorid enthielt.

### Beispiel 8

Zu 850 ml wasserfreier Fluorwasserstoffsäure wurden bei einer Innentemperatur zwischen 2 und 7°C im Verlaufe von 15 Minuten 1138 g des gemäß Beispiel 1 erhaltenen 3- und 4-Nitro-benzotrichlorid-Gemisches zugetropft. Danach wurde 17 Stunden bei Raumtemperatur und 8 Stunden bei 150°C und 25 bar Druck gerührt, dann auf Raumtemperatur abgekühlt und die überschüssige Fluorwasserstoffsäure destillativ entfernt. Der Rückstand wurde über eine Brücke destilliert und so wurden 695 g eines Gemisches aus 3- und 4-Nitrobenzotrifluorid (Reinheit 100 %, 76,8 % der Theorie) erhalten. Dieses wurde durch Feindestillation getrennt und so 521,6 g 3-Nitrobenzotrifluorid und 110,5 g 4-Nitrobenzotrifluorid mit einer Reinheit von jeweils über 99 % erhalten.

Analog und mit ähnlichen Ausbeuten wurden die gemäß den Beispielen 2 bis 4 erhaltenen Nitrobenzotrichlorid-Gemische fluoriert und durch Destillation getrennt.

### Beispiel 9

406 g eines gemäß Beispiel 8 (vor der Feindestillation) erhaltenen 3- und 4-Nitrobenzotrifluorid-Gemisches und 44 g Raney-Nickel wurden in 41 Methanol vorgelegt und zunächst 65 Stunden bei 20°C 5 bar, dann 40 Stunden bei 20°C 10 bar Wasserstoff aufgedrückt. Danach wurde das Reaktionsgemisch filtriert und das Filtrat eingedampft. Das so erhaltene Produkt (330 g, Reinheit 87,6 %, 84,5 % der Theorie) enthielt 78 % 3- und 22 % 4-Aminobenzotrifluorid.

### Beispiel 10

110 g eines gemäß Beispiel 8 (nach der Feindestillation) erhaltenen 4-Nitrobenzotrifluorids (Reinheit über 99 %) und 10 g Raney-Nickel wurden in 1 l Methanol vorgelegt und 40 Stunden bei 20°C 10 bar Wasserstoff aufgedrückt. Danach wurde das Reaktionsgemisch filtriert und das Filtrat eingedampft. So wurden 80,0 g (= 86,3 % d.Th.) 4-Trifluormethylanilin in einer Reinheit von über 99 % erhalten.

## Patentansprüche

1. Verfahren zur Herstellung von Trifluormethylanilinen der Formel (I) 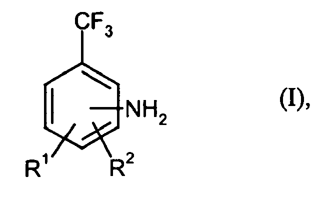 in der
R¹ für Wasserstoff, Fluor, Chlor, Brom, Methyl, Monochlormethyl, Dichlormethyl oder Formyl und
R² für Wasserstoff, Fluor oder Chlor stehen
und sich im Falle R¹ = R² = Wasserstoff die Aminogruppe in para-Position zur Trifluormethylgruppe befindet,
wobei man ein Benzotrichlorid der Formel (II) 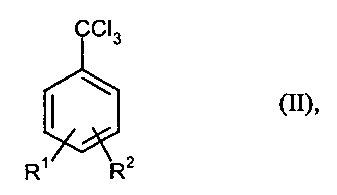 in der
R¹ und R² die bei Formel (I) angegebene Bedeutung haben,
nitriert, bei den so erhältlichen Nitrobenzotrichloriden die Trichlormethylgruppen durch Umsetzung mit wasserfreier Fluorwasserstoffsäure in Trifluormethylgruppen überführt und abschließend die Nitrogruppen reduziert, **dadurch gekennzeichnet, dass** die erhaltenen Verbindungen der Formel (I) mindestens 19 % Trifluormethylaniline enthalten, worin sich die Aminogruppe in para-Position zur Trifluormethylgruppe befindet.

2. Verfahren nach Anspruch 1, **dadurch gekennzeichnet, dass** in den Formeln (I) und (II) R¹ für Wasserstoff, Fluor oder Chlor und R² für Wasserstoff oder Chlor steht.

3. Verfahren nach Ansprüchen 1 und 2, **dadurch gekennzeichnet, dass** man in die Nitrierung Gemische aus Salpetersäure und Schwefelsäure einsetzt, wobei die Schwefelsäure mindestens 96 %ig ist und es sich bei der Salpetersäure um solche mit Gehalten um 100 % HNO₃ handelt.

4. Verfahren nach Anspruch 3, **dadurch gekennzeichnet, dass** man 1,2 bis 5 Mol Salpetersäure pro Mol Benzotrichlorid der Formel (II) einsetzt.

5. Verfahren nach Ansprüchen 3 und 4, **dadurch gekennzeichnet, dass** das Gewichtsverhältnis von Salpetersäure zu Schwefelsäure 0,5 bis 2:1 beträgt und die Nitrierung im Bereich -15 bis +50°C durchgeführt wird.

6. Verfahren nach Ansprüchen 1 bis 5, **dadurch gekennzeichnet, dass** man pro Mol Nitrobenzotrichlorid 3 bis 50 Mol wasserfreie Fluorwasserstoffsäure einsetzt, die Fluorierung bei Temperaturen im Bereich 0 bis 180°C und Drucken im Bereich 1 bis 5 bar durchführt.

7. Verfahren nach Ansprüchen 1 bis 6, **dadurch gekennzeichnet, dass** man auf der Stufe der Nitrobenzotrifluoride eine Isomerentrennung durchführt.

8. Verfahren nach Ansprüchen 1 bis 7, **dadurch gekennzeichnet, dass** man die Reduktion als katalytische Reduktion mit Wasserstoff in Gegenwart von Raney-Nickel oder Palladium enthaltenden Katalysatoren durchführt.

9. Verfahren nach Ansprüchen 1 bis 8, **dadurch gekennzeichnet, dass** man die Reduktion bei Temperaturen im Bereich von 20 bis 100°C durchführt.

10. Verfahren nach Ansprüchen 1 bis 9, **dadurch gekennzeichnet, dass** man die Reduktion bei Drucken im Bereich 1 bis 50 bar durchführt.

## Claims

1. Process for preparing trifluoromethylanilines of the formula (I) 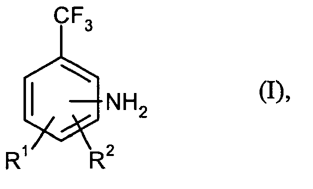 where
R¹ is hydrogen, fluorine, chlorine, bromine, methyl, monochloromethyl, dichloromethyl or formyl and
R² is hydrogen, fluorine or chlorine
and for R¹ = R² = hydrogen the amino group is para to the trifluoromethyl group,
in which a benzotrichloride of the formula (II) 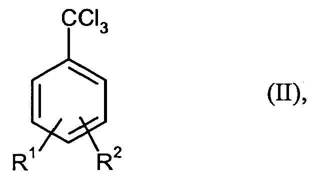 where
R¹ and R² are each as defined for the formula (I),
is nitrated and, in the nitrobenzotrichlorides thus obtainable, the trichloromethyl groups are converted into trifluoromethyl groups by reaction with anhydrous hydrofluoric acid and finally the nitro groups are reduced, **characterized in that** the compounds obtained of the formula (I) include at least 19% of trifluoromethylanilines in which the amino group is para to the trifluoromethyl group.

2. Process according to Claim 1, **characterized in that** in the formulae (I) and (II) R¹ is hydrogen, fluorine or chlorine and R² is hydrogen or chlorine.

3. Process according to Claims 1 and 2, **characterized in that** the benzotrichloride is nitrated using mixtures of nitric acid and sulphuric acid, the sulphuric acid being at least 96% strength and the nitric acid containing around 100% HNO₃.

4. Process according to Claim 3, **characterized in that** from 1.2 to 5 mol of nitric acid are used per mole of benzotrichloride of the formula (II).

5. Process according to Claims 3 and 4, **characterized in that** the weight ratio of nitric acid to sulphuric acid is 0.5 to 2:1 and the nitration is carried out in the range -15 to +50°C.

6. Process according to Claims 1 to 5, **characterized in that** 3 to 50 mol of anhydrous hydrofluoric acid are used per mole of nitrobenzotrichloride and the fluorination is carried out at temperatures in the range 0 to 180°C and pressures in the range 1 to 5 bar.

7. Process according to Claims 1 to 6, **characterized in that** an isomer separation is carried out at the nitrobenzotrifluoride stage.

8. Process according to Claims 1 to 7, **characterized in that** the reduction is carried out as a catalytic reduction with hydrogen in the presence of Raney nickel or palladium catalysts.

9. Process according to Claims 1 to 8, **characterized in that** the reduction is carried out at temperatures in the range from 20 to 100°C.

10. Process according to Claims 1 to 9, **characterized in that** the reduction is carried out at pressures in the range 1 to 50 bar.

## Revendications

1. Procédé de préparation de trifluorométhylanilines de formule (I) 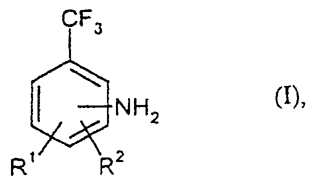 dans laquelle:
R¹ représente un atome d'hydrogène, un atome de fluor, un atome de chlore, un atome de brome, un groupe méthyle, un groupe monochlorométhyle, un groupe dichlorométhyle ou un groupe formyle ; et
R² représente un atome d'hydrogène, un atome de fluor ou un atome de chlore,
et au cas où R¹ = R² = un atome d'hydrogène, le groupe amino se trouve en position para par rapport au groupe trifluorométhyle,
dans lequel un benzotrichlorure de formule (II) 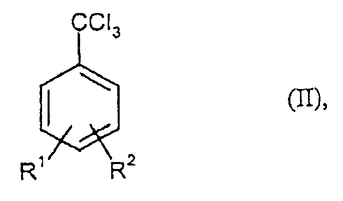 dans laquelle :
R¹ et R² présentent la signification indiquée pour la formule (I),
est nitré et, dans les nitrobenzotrichlorures pouvant ainsi être obtenus, les groupes trichlorométhyles sont transformés en groupes trifluorométhyles par réaction avec de l'acide fluorhydrique anhydre, et les groupes nitro sont enfin réduits, **caractérisé en ce que** les composés obtenus de formule (I) comprennent au moins 19 % de trifluorométhylanilines, dans lesquelles le groupe amino se trouve en position para par rapport au groupe trifluorométhyle.

2. Procédé selon la revendication 1, **caractérisé en ce que**, dans les formules (I) et (II), R¹ représente un atome d'hydrogène, un atome de fluor ou un atome de chlore, et R² représente un atome d'hydrogène ou un atome de chlore.

3. Procédé selon les revendications 1 et 2, **caractérisé en ce que**, lors de la nitration, on utilise des mélanges d'acide nitrique et d'acide sulfurique, l'acide sulfurique étant à au moins 96 % et l'acide nitrique présentant des teneurs d'environ 100 % de HNO₃.

4. Procédé selon la revendication 3, **caractérisé en ce que** l'on utilise de 1,2 à 5 mol d'acide nitrique par mole de benzotrichlorure de formule (II).

5. Procédé selon les revendications 3 et 4, **caractérisé en ce que** le rapport pondéral entre l'acide nitrique et l'acide sulfurique est de 0,5 à 2:1, et la nitration est réalisée dans la plage de - 15 à + 50°C.

6. Procédé selon les revendications 1 à 5, **caractérisé en ce que** l'on utilise, par mole de nitrobenzotrichlorure, de 3 à 50 mol d'acide fluorhydrique anhydre, et la fluoration est réalisée à des températures dans la plage de 0 à 180°C et à des pressions dans la plage de 1 à 5 bar.

7. Procédé selon les revendications 1 à 6, **caractérisé en ce qu'**une séparation des isomères est réalisée au stade des nitrobenzotrifluorures.

8. Procédé selon les revendications 1 à 7, **caractérisé en ce que** la réduction est réalisée en tant que réduction catalytique avec de l'hydrogène en présence de nickel de Raney ou de catalyseurs contenant du palladium.

9. Procédé selon les revendications 1 à 8, **caractérisé en ce que** la réduction est réalisée à des températures dans la plage de 20 à 100°C.

10. Procédé selon les revendications 1 à 9, **caractérisé en ce que** la réduction est réalisée à des pressions dans la plage de 1 à 50 bar.
